# EUROPEAN PATENT APPLICATION

(11) **EP 3 450 975 A1**
(43) Date of publication of application: **06.03.2019**
(21) Application number: 17306145.8
(22) Date of filing: 05.09.2017
(51) Int. Cl.: G01N 33/18, G01N 27/416, G01N 35/00, G01N 35/10

(54) **PORTABLE SYSTEM FOR PHYSICOCHEMICAL ANALYSIS OF A SOIL FLUID**

(71) Applicant: METAGRITECH, 91000 Evry (FR)
(72) Inventor: FILS, Julien, 38210 Saint-Quentin-sur-Isère (FR)
(74) Representative: Icosa

(57) **Abstract**

The present invention relates to a portable system (1) for sampling and physicochemical analysis of a fluid, comprising a fluidic circuit, wherein the fluidic circuit comprises: a sampling module (2) configured to collect a sample of a fluid extracted from a soil; a disposable and removable cartridge (3) comprising a first storing chamber (4) filled with a first calibration liquid and a second storing chamber (5) filled with a second calibration liquid and further comprising a first measure chamber (7) comprising at least one disposable physicochemical sensor (8); a distribution module (9) in fluid communication with the sampling module (2) and the disposable and removable cartridge (3), wherein said distribution module (9) is capable of fluidly connect the first measure chamber (7) to one of the storing chambers (4, 5) or to the sampling module (2); and a pumping means to provide a flow from the sampling module (2) to the first measure chamber (7) and a flow from one of the storing chambers (4, 5) to the first measure chamber (7).

## Description

### FIELD OF INVENTION

The present invention pertains to the field of chemical analysis of liquid samples. In particular, the invention relates to a portable and easy to handle physiochemical analyzer configured to extract fluid samples from soil and analyze them in an automate routine.

### BACKGROUND OF INVENTION

Generally, chemical analyzers are apparatus for analyzing liquid samples which have been developed in various way to be adapted to different environments and for different purposes. Agricultural production requires a continuous and attentive monitoring of chemicals along their production chain. In general practice, fluid samples are directly collected or extracted from soil and analyzed in an equipped laboratory or automated analyzer, or more complex monitoring devices may be installed along pipe lines. Moreover, small producers usually do not own in their facilities those types of analysis devices and therefore they have to rely on external analysis laboratories to analyze their samples. However, collecting and transporting samples to a laboratory is a time-consuming process which does not allow a continuous monitoring in real time of the chemicals. In other cases, the online analysis with optical sensor or electrochemical sensors such as Ion selective electrode (ex: pH meter) requires to calibrate the sensors frequently which is also a time-consuming operation.

One of the object of the present invention is an automated sampling system coupled to an analyzer comprising multiple physicochemical sensors assembled in such a way to gather extremely reduced dimensions and therefore allow to a user an easy transport. The present invention discloses an improved system capable of automatically calibrating its chemical sensors before measurement, sampling a liquid and analyzing it on site.

### SUMMARY

The invention relates to a portable system for sampling and physicochemical analysis of a fluid, comprising a fluidic circuit, wherein the fluidic circuit comprises:
- a sampling module configured to collect a sample of a fluid extracted from a soil;
- a disposable and removable cartridge comprising a first storing chamber filled with a first calibration liquid and a second storing chamber filled with a second calibration liquid and further comprising a first measure chamber comprising at least one disposable physicochemical sensor;
- a distribution module (9) in fluid communication with the sampling module and the disposable and removable cartridge, wherein said distribution module (9) is capable of fluidly connect the first measure chamber to one of the storing chambers or to the sampling module; and
- a pumping means to provide a flow from the sampling module to the first measure chamber and a flow from one of the storing chambers to the first measure chamber.

According to one embodiment, the cartridge further comprises a third storing chamber comprising cleaning liquid and being in fluid communication with the distribution module; wherein the distribution module is capable of fluidly connecting the first measure chamber with said third storing chamber.

According to one embodiment, the portable system further comprises a second measure chamber comprising at least one physicochemical sensor and being in fluid communication with the sampling module and the distribution module; wherein the distribution module is capable of fluidly connect the second measure chamber with one of the storing chambers.

According to one embodiment, the first storing chambers, the second storing chamber and the third storing chamber are fluidly isolated from each other.

According to one embodiment, the sampling module comprises an inlet configured to be in fluid communication with at least one collection tube of a lysimeter.

According to one embodiment, the pumping means are fluidly connected to the sampling module, ensuring a fluid flow in at least fluidic circuit, the first measure chamber and the second measure chamber.

According to one embodiment, pumping means are fluidly connected to the storing chambers and capable of providing a flow from the storing chambers to the first measure chamber and second measure chamber and backwards.

According to one embodiment, the first calibration liquid and the second calibration liquid are solutions comprising a same chemical species with a significantly different concentration.

According to one embodiment, the at least one physicochemical sensor comprised in the second measure chamber is an optical sensor or an electrochemical sensor.

According to one embodiment, the at least one physicochemical sensor comprised in the second measure chamber is adapted to monitor the conductivity, the oxidation-reduction potential, the temperature of the fluid, chemical oxygen demand, the carbon dioxide concentration, total organic carbon and/or dissolved oxygen.

According to one embodiment, the portable system further comprising a communication module capable of transmitting information to an interface device.

According to one embodiment, the pumping means is a peristaltic pump.

According to one embodiment, the sampling module further comprises a UV emitting light source.

The present invention further relates to a method for sampling and physicochemical analyzing a fluid using the portable system as described in the embodiment hereabove.

The present invention further relates to a computer implemented method configured to communicate and command the portable system.

The present invention further relates to a measurement apparatus for sampling and physicochemical analysis of a fluid, comprising at least one portable system according to the embodiment hereabove, and at least one extracting means to extract a fluid sample from a soil, where the measurement apparatus comprises a junction element for exchanging a liquid.

The measurement apparatus comprises a frame comprising at least one first cavity for receiving at least one portable system and at least one second cavity for receiving at least one extracting means.

### DEFINITIONS

In the present invention, the following terms have the following meanings:
- As used herein the singular forms "**a**", "**an**", and "**the**" include plural reference unless the context clearly dictates otherwise.
- The term "**about**" is used herein to mean approximately, roughly, around, or in the region of. When the term "about" is used in conjunction with a numerical range, it modifies that range by extending the boundaries above and below the numerical values set forth. In general, the term "about" is used herein to modify a numerical value above and below the stated value by a variance of 20 percent, preferably of 5 percent.
- The term "**significantly**" is used herein to modify a numerical value above and below the stated value by a variance of more than 80 percent.
- "**Fluid**" refers to any substance that continually deforms (flows) under an applied shear stress. A fluid according to the present invention have densities ranging from 0.8 g/ml to 1.5 g/ml.
- "**Lysimeter**" refers to a system configured for extraction of fluid from a soil. The extraction may be obtained by creating a low pressure or by water evaporation. The lysimeter comprises a collection tube through which a sample of fluid extracted from the soil exit the lysimeter.
- "**Soil**" refers to a mixture of minerals, organic matter, gases, liquids, and countless organisms. Soil consists of a solid phase of minerals and organic matter (the soil matrix), as well as a porous phase that holds gases (the soil atmosphere) and water (the soil solution). A typical soil is about 50% solids (45% mineral and 5% organic matter), and 50% voids of which half is occupied by water and half by gas.

### DETAILED DESCRIPTION

The following detailed description will be better understood when read in conjunction with the drawings. For the purpose of illustrating, the device is shown in the preferred embodiments. It should be understood, however that the application is not limited to the precise arrangements, structures, features, embodiments, and aspect shown. The drawings are not drawn to scale and are not intended to limit the scope of the claims to the embodiments depicted. Accordingly, it should be understood that where features mentioned in the appended claims are followed by reference signs, such signs are included solely for the purpose of enhancing the intelligibility of the claims and are in no way limiting on the scope of the claims.

This invention relates to a portable system **1** for sampling and physicochemical analysis of a fluid extracted from a soil and which comprises a fluidic circuit.

According to one embodiment, said fluidic circuit comprises a sampling module **2,** a disposable and removable cartridge **3,** enclosing physicochemical sensors **8** and at least storing chambers (**4, 5**) with calibration liquids, a distribution module **9** and a pumping means; where the sampling module **2,** the distribution module **9** and the pumping means are comprised in a same main body of the portable system.

### SAMPLING MODULE

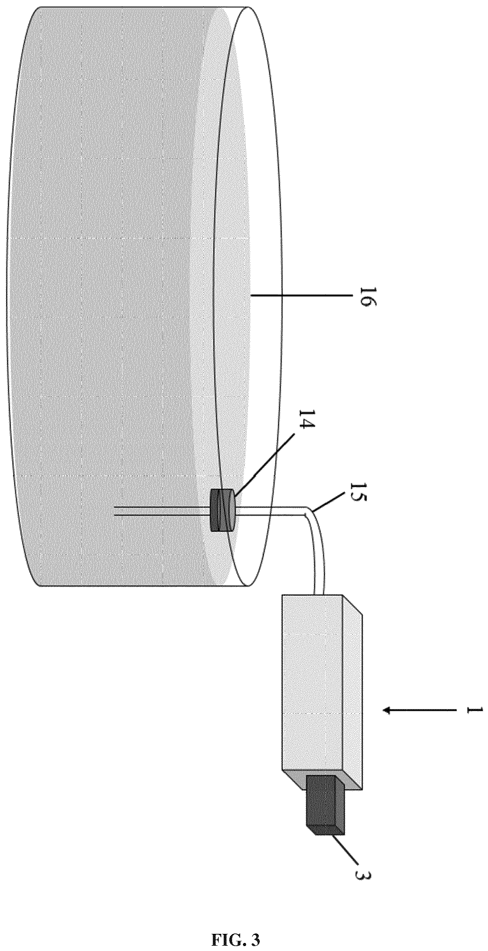

According to one embodiment, the sampling module **2** is configured to collect a sample of a fluid extracted from a soil. The sampling module **2** may comprise an inlet **11** configured to be in fluid communication with at least one collection tube of at least one lysimeter **16,** as shown in Figure **3****.** The inlet **11** may be fluidly connected to the collection tube of the lysimeter **16** through a communication tube **14** of variable length, preferably ranging from about 1 cm to about 5 m. According to one embodiment, the inlet **11** may be connected to multiple collection tube associated to multiple lysimeter **16** inserted in the soil **15** at different depth. The lysimeter **16** may be a suction lysimeter, simple chamber lysimeter, dual chamber lysimeter, pan lysimeter, static tension lysimeter, controlled tension lysimeter or a weighting lysimeter. The lysimeter **16** used may be configure to collect a water same at a depth ranging from 10 cm to 2 m. The fluid may be extracted from the soil with any means known by the man skilled in the art.

According to one embodiment, the sampling module **2** comprises a sampling pumping means to transfer the soil extracted fluid sample from the collection tube of the lysimeter **16** to the inlet **11** and therefore inside the fluidic circuit. The sampling pumping means may be any type of mechanical pumping means know by the man skilled in the art, and preferably a peristaltic pump.

According to an alternative embodiment, the portable system **1** is mechanically connected to the lysimeter **16** and the inlet **11** is in fluidic communication with the collection tube of at least one lysimeter **16.** The portable system **1** may be configured to be inserted inside the lysimeter **16.**

According to one embodiment, the sampling module **2** further comprises a UV emitting light source **13.** The UV light permits to kill the eventual cells entering by the inlet and therefore to prevent the proliferation of said cells inside the portable system **1.** Cells proliferation would indeed compromise the working principles of the portable system **1.**

According to one embodiment, the main body of the portable system **1** is a cuboid. The main body may have a height comprised between 1 cm and 2 m, preferably a height 5 cm, 6 cm, 7 cm, 8 cm, 9 cm, 10 cm, 11 cm, 12 cm, 13 cm, 14 cm, 15 cm, 16 cm, 17 cm, 18 cm, 19 cm, 20 cm, 21 cm, 22 cm, 23 cm, 24 cm, 25 cm, 26 cm, 27 cm, 28 cm, 29 cm or 30 cm, a width comprised between 1 cm and 2 m, preferably a width of 5 cm, 6 cm, 7 cm, 8 cm, 9 cm, 10 cm, 11 cm, 12 cm, 13 cm, 14 cm, 15 cm, 16 cm, 17 cm, 18 cm, 19 cm, 20 cm, 21 cm, 22 cm, 23 cm, 24 cm, 25 cm, 26 cm, 27 cm, 28 cm, 29 cm or 30 cm, and a length comprised between 1 cm and 2 m, preferably a length of 20 cm, 21 cm, 22 cm, 23 cm, 24 cm, 25 cm, 26 cm, 27 cm, 28 cm, 29 cm, 30 cm 31 cm, 32 cm, 33 cm, 34 cm, 35 cm, 36 cm, 37 cm, 38 cm, 39 cm, 40 cm, 41 cm, 42 cm, 43 cm, 44 cm, 45 cm, 46 cm, 47 cm, 48 cm, 49 cm or 50 cm.

### CARTRIDGE

The disposable and removable cartridge **3** is configured to be easily plugged in the main body of the portable system **1,** removed and thrown away after a period of use. Said period of use depends on the measurement frequency and may range from 1 week to 6 months, preferably from 1 week to 5 months, from 1 week to 4 months, from 1 week to 3 months or from 1 week to 2 months. The cartridge **3** may comprise a first retaining means configured to cooperate with a second retaining means comprised in the main body of the portable system.

According to an alternative embodiment, the cartridge **3** is configured to be directly inserted in the lysimeter **16.**

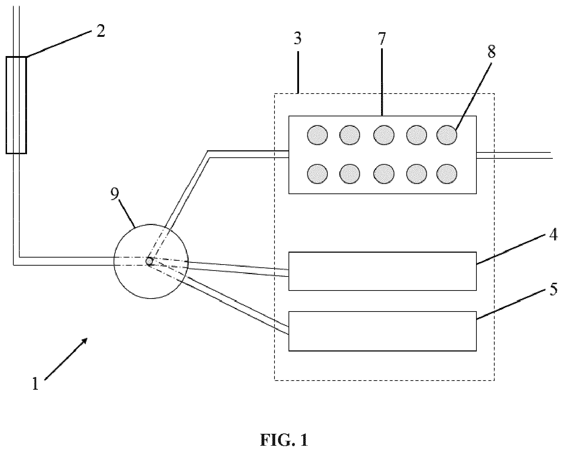

According to one embodiment, the disposable and removable cartridge **3** comprises a first measure chamber **7** comprising at least one disposable physicochemical sensor **8,** as shown in Figure **1****.** The at least one physiochemical sensor **8** may be a chemical sensor, preferably an electrochemical sensor (such as but not limited to amperometric, potentiometric, voltametric or impedancemetric sensors) or an ion-selective electrode comprising glass membranes, crystalline membranes or ion-exchange resin membranes. The at least one physiochemical sensor **8** may be configured to detect the concentration of at least one ion selected in the group of, but not limited to: K⁺, Na⁺, Ca²⁺, Mg²⁺, Cl⁻, NH₄⁺, NO₂⁻, NO₃⁻, PO₄3⁻ and the like. The at least one physicochemical sensor **8** may be a pH meter. According to one embodiment, the first measuring chamber **7** comprises at least 1, 2, 3, 4, 5, 6, 7, 8, 9 or 10 disposable physicochemical sensors **8.**

According to one embodiment, the disposable and removable cartridge **3** comprises a first storing chamber **4** filled with a first calibration liquid and a second storing chamber **5** filled with a second calibration liquid. Said calibration liquids are used during the calibration of the physicochemical sensors of the portable system **1.**

According to one embodiment, the first calibration liquid and the second calibration liquid are solutions comprising a same chemical species with a significantly different concentration. The first calibration liquid and the second calibration liquid may comprise at least one chemical species selected in the group of, but not limited to: K⁺, Na⁺, Ca²⁺, Mg²⁺, Cl⁻, NH₄⁺, NO₂⁻, NO₃⁻, PO₄³⁻ and the like.

The concentration of the chemical species K⁺ in the first calibration liquid may be 0.05 mg/L, 0.1 mg/L, 0.15 mg/L, 0.20 mg/L, 0.25 mg/L, 0.30 mg/L, 0.35 mg/L, 0.40 mg/L, 0.45 mg/L, 0.50 mg/L, 0.55 mg/L or 0.60 mg/L. The concentration of the chemical species K⁺ in the second calibration liquid may be 7000 mg/L, 7100 mg/L, 7200 mg/L, 7300 mg/L, 7500 mg/L, 7600 mg/L, 7700 mg/L, 7800 mg/L, 7900 mg/L, 8000 mg/L, 8500 mg/L, 9000 mg/L, 9500 mg/L or 10000 mg/L. The concentration of the chemical species Na⁺ in the first calibration liquid may be 0.05 mg/L, 0.06 mg/L, 0.07 mg/L, 0.08 mg/L, 0.09 mg/L, 0.1 mg/L, 0.15 mg/L, 0.20 mg/L, 0.25 mg/L, 0.30 mg/L, 0.35 mg/L, 0.40 mg/L, 0.45 mg/L, 0.50 mg/L, 0.55 mg/L or 0.60 mg/L. The concentration of the chemical species Na⁺ in the second calibration liquid may be 6000 mg/L, 6100 mg/L, 6200 mg/L, 6300 mg/L, 6500 mg/L, 6600 mg/L, 6700 mg/L, 6800 mg/L, 6900 mg/L, 7000 mg/L, 7500 mg/L, 8000 mg/L, 8500 mg/L or 9000 mg/L. The concentration of the chemical species Ca²⁺ in the first calibration liquid may be 0.05 mg/L, 0.1 mg/L, 0.15 mg/L, 0.20 mg/L, 0.25 mg/L, 0.30 mg/L, 0.35 mg/L, 0.40 mg/L, 0.45 mg/L, 0.50 mg/L, 0.55 mg/L or 0.60 mg/L. The concentration of the chemical species Ca²⁺ in the second calibration liquid may be 3000 mg/L, 3100 mg/L, 3200 mg/L, 3300 mg/L, 3500 mg/L, 3600 mg/L, 3700 mg/L, 3800 mg/L, 3900 mg/L, 4000 mg/L, 4500 mg/L, 5000 mg/L, 5500 mg/L or 6000 mg/L. The concentration of the chemical species Mg²⁺ in the first calibration liquid may be 0.05 mg/L, 0.1 mg/L, 0.15 mg/L, 0.20 mg/L, 0.25 mg/L, 0.30 mg/L, 0.35 mg/L, 0.40 mg/L, 0.45 mg/L, 0.50 mg/L, 0.55 mg/L or 0.60 mg/L. The concentration of the chemical species Mg²⁺ in the second calibration liquid may be 2000 mg/L, 2100 mg/L, 2200 mg/L, 2300 mg/L, 2500 mg/L, 2600 mg/L, 2700 mg/L, 2800 mg/L, 2900 mg/L, 3000 mg/L, 3500 mg/L, 4000 mg/L, 4500 mg/L or 5000 mg/L. The concentration of the chemical species Cl⁻ in the first calibration liquid may be 0.05 mg/L, 0.1 mg/L, 0.15 mg/L, 0.20 mg/L, 0.25 mg/L, 0.30 mg/L, 0.35 mg/L, 0.40 mg/L, 0.45 mg/L, 0.50 mg/L, 0.55 mg/L or 0.60 mg/L. The concentration of the chemical species Cl⁻ in the second calibration liquid may be 8000 mg/L, 8500 mg/L, 9000 mg/L, 9500 mg/L, 10000 mg/L, 10500 mg/L, 11000 mg/L, 11500 mg/L or 12000 mg/L. The concentration of the chemical species NH₄⁺ in the first calibration liquid may be 0.01 mg/L, 0.02 mg/L, 0.03 mg/L, 0.04 mg/L, 0.05 mg/L, 0.06 mg/L, 0.07 mg/L, 0.08 mg/L, 0.09 mg/L, 0.1 mg/L, 0.15 mg/L, 0.20 mg/L, 0.25 mg/L, 0.30 mg/L, 0.35 mg/L, 0.40 mg/L, 0.45 mg/L, 0.50 mg/L, 0.55 mg/L or 0.60 mg/L. The concentration of the chemical species NH₄⁺ in the second calibration liquid may be 500 mg/L, 600 mg/L, 700 mg/L, 800 mg/L, 900 mg/L, 1000 mg/L, 1100 mg/L, 1200 mg/L, 1300 mg/L, 1400 mg/L, 1500 mg/L, 2000 mg/L, 2500 mg/L or 3000 mg/L. The concentration of the chemical species NO₂⁻ in the first calibration liquid may be 0.05 mg/L, 0.06 mg/L, 0.07 mg/L, 0.08 mg/L, 0.09 mg/L, 0.1 mg/L, 0.15 mg/L, 0.20 mg/L, 0.25 mg/L, 0.30 mg/L, 0.35 mg/L, 0.40 mg/L, 0.45 mg/L, 0.50 mg/L, 0.55 mg/L or 0.60 mg/L. The concentration of the chemical species NO₂⁻ in the second calibration liquid may be 5500 mg/L, 5600 mg/L, 5700 mg/L, 5800 mg/L, 5900 mg/L, 6000 mg/L, 6100 mg/L, 6200 mg/L, 6300 mg/L, 6400 mg/L, 6500 mg/L, 7000 mg/L, 7500 mg/L or 8000 mg/L. The concentration of the chemical species NO₃⁻ in the first calibration liquid may be 0.05 mg/L, 0.06 mg/L, 0.07 mg/L, 0.08 mg/L, 0.09 mg/L, 0.1 mg/L, 0.15 mg/L, 0.20 mg/L, 0.25 mg/L, 0.30 mg/L, 0.35 mg/L, 0.40 mg/L, 0.45 mg/L, 0.50 mg/L, 0.55 mg/L or 0.60 mg/L. The concentration of the chemical species NO₃⁻ in the second calibration liquid may be 5500 mg/L, 5600 mg/L, 5700 mg/L, 5800 mg/L, 5900 mg/L, 6000 mg/L, 6100 mg/L, 6200 mg/L, 6300 mg/L, 6400 mg/L, 6500 mg/L, 7000 mg/L, 7500 mg/L or 8000 mg/L. The concentration of the chemical species PO₄³⁻ in the first calibration liquid may be 0.05 mg/L, 0.1 mg/L, 0.15 mg/L, 0.20 mg/L, 0.25 mg/L, 0.30 mg/L, 0.35 mg/L, 0.40 mg/L, 0.45 mg/L, 0.50 mg/L, 0.55 mg/L or 0.60 mg/L. The concentration of the chemical species PO₄³⁻ in the second calibration liquid may be 8000 mg/L, 8500 mg/L, 9000 mg/L, 9500 mg/L, 10000 mg/L, 10500 mg/L, 11000 mg/L, 11500 mg/L or 12000 mg/L. The pH of the first calibration liquid may be a pH 0, pH 0.5, pH 1, pH 1.5, pH 2, pH 2.5 or pH 3. The pH of the second calibration liquid may be a pH 10, pH 10.5, pH 11, pH 11.5, pH 12, pH 12.5, pH 13, Ph 13.5 or pH 14.

The concentrations of the chemical species in the first calibration liquid and the second calibration liquid are known by the user. This information may be written on the cartridge in the form of a bar code or alphanumeric code or alternatively registered in a Near Field communication device comprised in the cartridge.

At least two calibration liquids are needed to properly calculate a calibration curve for the physicochemical sensors. According to one embodiment, the cartridge further comprises complementary storage chambers comprising complementary calibration liquids, allowing to obtain a more accurate calibration curve. A complementary storage chamber associated to the first and second storage chamber (**4, 5**) may comprise a calibration liquid comprising the same chemical species of the first and second storage chamber (**4, 5**) with a concentration comprised in the range of concentration defined by the concentrations of the first and second calibration liquid.

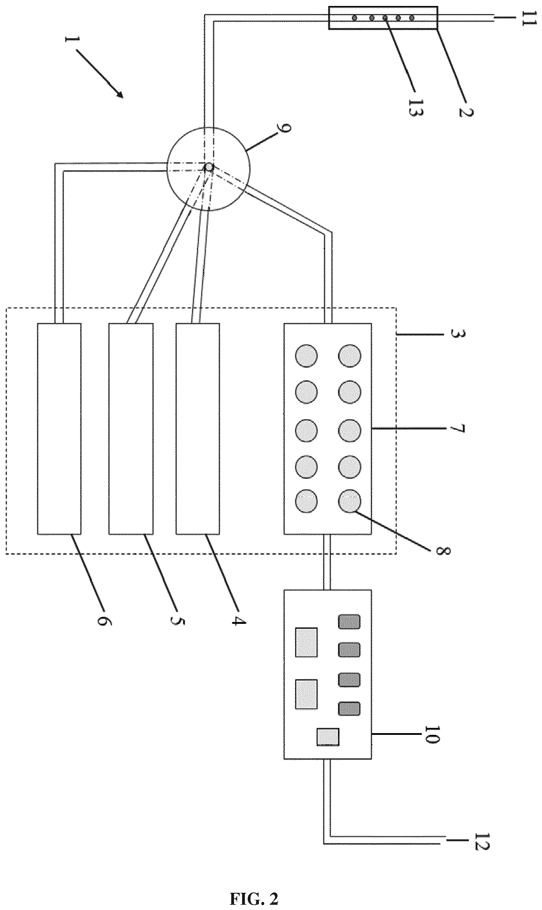

According to one embodiment with reference to Figure **2****,** the cartridge **3** further comprises a third storing chamber **6** in fluid communication with the distribution module **9** and comprising a cleaning liquid. The cleaning liquid is a solution that may be water or any solution known from the man skilled in the art to clean a fluidic circuit and physicochemical sensors.

According to preferred embodiment, the cartridge **3** comprises a total of 5 storing chambers: the first and second storing chamber (**4, 5**) comprising calibration liquids with a first chemical species in two different concentrations, the third storing chamber **6** comprising the cleaning liquid, and a fourth and fifth storing chambers comprising calibration liquids with a second chemical species in two different concentrations.

According to one embodiment, the first storing chamber **4,** the second storing chamber **5** and the third storing chamber **6** are fluidly isolated from each other.

According to one embodiment, the cartridge is recyclable. The calibration liquids and the cleaning liquid comprised respectively in the first storing chamber **4,** the second storing chamber **5** and the third storing chamber **6** may be replaced after a first use. The disposable physicochemical sensors **8** comprised in the first measure chamber **7** may be replaced by new ones.

According to an alternative embodiment, the cartridge **3** is cuboid. The cartridge **3** may have a height ranging from 1 cm to 1 m, preferably a height of 1 cm, 1.1 cm, 1.2 cm, 1.3 cm, 1.4 cm, 1.5 cm, 1.6 cm, 1.7 cm, 1.8 cm, 1.9 cm, 2 cm, 2.1 cm, 2.2 cm, 2.3 cm, 2.4 cm, 2.5 cm, 2.6 cm, 2.7 cm, 2.8 cm, 2.9 cm, 3 cm, 3.1 cm, 3.2 cm, 3.3 cm, 3.4 cm, 3.5 cm, 3.6 cm, 3.7 cm, 3.8 cm, 3.9 cm, 4 cm, 4.1 cm, 4.2 cm, 4.3 cm, 4.4 cm, 4.5 cm, 4.6 cm, 4.7 cm, 4.8 cm, 4.9 cm, 5 cm, 5.1 cm, 5.2 cm, 5.3 cm, 5.4 cm, 5.5 cm, 5.6 cm, 5.7 cm, 5.8 cm, 5.9 cm, 6 cm, 6.1 cm, 6.2 cm, 6.3 cm, 6.4 cm, 6.5 cm, 6.6 cm, 6.7 cm, 6.8 cm, 6.9 cm, 7 cm, 7.1 cm, 7.2 cm, 7.3 cm, 7.4 cm, 7.5 cm, 7.6 cm, 7.7 cm, 7.8 cm, 7.9 cm, 8 cm, 8.1 cm, 8.2 cm, 8.3 cm, 8.4 cm, 8.5 cm, 8.6 cm, 8.7 cm, 8.8 cm, 8.9 cm, 9 cm, 9.1 cm, 9.2 cm, 9.3 cm, 9.4 cm, 9.5 cm, 9.6 cm, 9.7 cm, 9.8 cm, 9.9 cm, 10 cm, 11 cm, 12 cm or 13 cm, a width ranging from 1 cm to 1 m, preferably a width of 1 cm, 1.1 cm, 1.2 cm, 1.3 cm, 1.4 cm, 1.5 cm, 1.6 cm, 1.7 cm, 1.8 cm, 1.9 cm, 2 cm, 2.1 cm, 2.2 cm, 2.3 cm, 2.4 cm, 2.5 cm, 2.6 cm, 2.7 cm, 2.8 cm, 2.9 cm, 3 cm, 3.1 cm, 3.2 cm, 3.3 cm, 3.4 cm, 3.5 cm, 3.6 cm, 3.7 cm, 3.8 cm, 3.9 cm, 4 cm, 4.1 cm, 4.2 cm, 4.3 cm, 4.4 cm, 4.5 cm, 4.6 cm, 4.7 cm, 4.8 cm, 4.9 cm, 5 cm, 5.1 cm, 5.2 cm, 5.3 cm, 5.4 cm, 5.5 cm, 5.6 cm, 5.7 cm, 5.8 cm, 5.9 cm, 6 cm, 6.1 cm, 6.2 cm, 6.3 cm, 6.4 cm, 6.5 cm, 6.6 cm, 6.7 cm, 6.8 cm, 6.9 cm, 7 cm, 7.1 cm, 7.2 cm, 7.3 cm, 7.4 cm, 7.5 cm, 7.6 cm, 7.7 cm, 7.8 cm, 7.9 cm, 8 cm, 8.1 cm, 8.2 cm, 8.3 cm, 8.4 cm, 8.5 cm, 8.6 cm, 8.7 cm, 8.8 cm, 8.9 cm, 9 cm, 9.1 cm, 9.2 cm, 9.3 cm, 9.4 cm, 9.5 cm, 9.6 cm, 9.7 cm, 9.8 cm, 9.9 cm, 10 cm, 11 cm, 12 cm or 13 cm and a length ranging from 1 cm to 1 m, preferably a length of 1 cm, 1.1 cm, 1.2 cm, 1.3 cm, 1.4 cm, 1.5 cm, 1.6 cm, 1.7 cm, 1.8 cm, 1.9 cm, 2 cm, 2.1 cm, 2.2 cm, 2.3 cm, 2.4 cm, 2.5 cm, 2.6 cm, 2.7 cm, 2.8 cm, 2.9 cm, 3 cm, 3.1 cm, 3.2 cm, 3.3 cm, 3.4 cm, 3.5 cm, 3.6 cm, 3.7 cm, 3.8 cm, 3.9 cm, 4 cm, 4.1 cm, 4.2 cm, 4.3 cm, 4.4 cm, 4.5 cm, 4.6 cm, 4.7 cm, 4.8 cm, 4.9 cm, 5 cm, 5.1 cm, 5.2 cm, 5.3 cm, 5.4 cm, 5.5 cm, 5.6 cm, 5.7 cm, 5.8 cm, 5.9 cm, 6 cm, 6.1 cm, 6.2 cm, 6.3 cm, 6.4 cm, 6.5 cm, 6.6 cm, 6.7 cm, 6.8 cm, 6.9 cm, 7 cm, 7.1 cm, 7.2 cm, 7.3 cm, 7.4 cm, 7.5 cm, 7.6 cm, 7.7 cm, 7.8 cm, 7.9 cm, 8 cm, 8.1 cm, 8.2 cm, 8.3 cm, 8.4 cm, 8.5 cm, 8.6 cm, 8.7 cm, 8.8 cm, 8.9 cm, 9 cm, 9.1 cm, 9.2 cm, 9.3 cm, 9.4 cm, 9.5 cm, 9.6 cm, 9.7 cm, 9.8 cm, 9.9 cm, 10 cm, 11 cm, 12 cm or 13 cm. The cartridge **3** may have any shape and dimensions that the man skilled in the art would consider suitable for the application at the present invention.

According to an alternative embodiment, the cartridge **3** comprises two separate parts: a first cartridge and a second cartridge. According this embodiment, the first cartridge comprises 2, 3, 4, 5, 6, 7, 8, 9, 10, 11, 12 or 13 storing chambers, wherein at least one of said storing chambers comprises a cleaning liquid and the others contains calibration liquids. According to this embodiment, the second cartridge comprises at least one measuring chamber comprising at least one physicochemical sensor. According to this embodiment, the first and the second cartridge are independently removable from the main body of the portable system **1.**

### DISTRIBUTION MODULE

According to one embodiment, the distribution module **9** is in fluid communication with the sampling module **2** and the disposable and removable cartridge **3,** wherein said distribution module **9** is capable of fluidly connecting the first measure chamber **7** to one of the first storing chamber **4,** second storing chamber **5,** third storing chamber **6** or sampling module **2.** The distribution module **9** may comprise multiple valves, preferably at least 4. The distribution module **9** may comprise 4, 5, 6, 7, 8, 9, 10, 11, 12, 13, 14, 15, 16, 17, 18, 19, 20, 25 or 30 valves. Alternatively, the distribution module **9** may be a rotative distributor.

### SECOND MEASURE CHAMBER

According to one embodiment, the portable system **1** further comprises in the main body a second measure chamber **10.** According to this embodiment, the second measure chamber **10** comprises at least one physicochemical sensor **8,** preferably non-disposable, which is in fluid communication with the sampling module **2** and the distribution module **9.** According to this embodiment, the distribution module **9** is capable of fluidly connecting the second measure chamber **10** with each of storing chambers (**4**, **5**, **6**).

According to one embodiment, the at least one physicochemical sensor **8** comprised in the second measure chamber **10** is an optical sensor or an electrochemical sensor. The optical chemical sensor may be configured to use colorimetry, light absorption and luminescence. The at least one physicochemical sensor **8** comprised in the second measure chamber **10** may be adapted to monitor the conductivity, the oxidation-reduction potential and/or the temperature of the fluid. According to one embodiment, the at least one physicochemical sensor **8** may be a sensor to measure the chemical oxygen demand, the carbon dioxide concentration, the dissolved oxygen, total organic carbon or the water turbidity.

Those types of physicochemical sensors are robust and do not need to be replaced as frequently as the physicochemical sensors comprised in the cartridge **3,** and therefore are installed in the portable system main body.

Each of the first storing chamber **4,** the second storing chamber **5** and the third storing chamber **6** should have a sufficient to store a volume of liquid capable to cover all the physicochemical sensors **8** when said liquid has been completely transferred in the first and second measure chambers (**4**, **5**, **6**). Preferably, the liquid volume should cover at least approximately 3 mm of the active area of the physicochemical sensors **8.** The volume of the storing chambers may be of 100 ml, 200 ml, 300 ml, 350 ml, 400 ml, 450 ml, 500 ml, 550 ml, 600 ml, 650 ml, 700 ml, 750 ml, 800 ml, 850 ml, 900 ml, 950 ml, 1000 ml, 1500 ml, 2000 ml, 2500 ml or 3000 ml.

According to one embodiment, the portable system **1** further comprises a displacement means to displace the physicochemical sensors **8** comprised in the first and second measuring chamber (**7**,**10**) in proximity of the storing chambers (**4**, **5**, **6**) and to be in fluid communication with said storing chambers (**4**, **5**, **6**). The displacement means may further displace the physicochemical sensors **8** comprised in the first and second measuring chamber (**7**, **10**) to be in fluid communication with the inlet **11** to perform the measurements. The displacement means may be may means of no-limiting example a sprocket or a wheel.

### PUMPING MEANS

According to one embodiment, the pumping means are configured to provide a flow from the sampling module **2** to the first measure chamber **7.** According to one embodiment, the pumping means are fluidly connected to the sampling module **2,** ensuring a fluid flow at least in the fluidic circuit, the first measure chamber **7** and the second measure chamber **10.** According to one embodiment, the pumping means are further configured to provide a flow from the first storing chamber **4,** the second storing chamber **5,** the third storing chamber **6** and/or the sampling module **2** to the first measure chamber **7.** The pumping means may be chosen from, but not limited to: peristaltic pump, rotary lobe pump, progressive cavity pump, rotary gear pump, piston pump, diaphragm pump, screw pump, gear pump, hydraulic pump, rotary vane pump, peristaltic pump, flexible impeller pump and the like.

According to one embodiment, the pumping means, fluidly connected to the first storing chamber **4,** the second storing chamber **5,** the third storing chamber **6,** are capable of providing a flow from said storing chambers (**4**, **5**, **6**) to the first measure chamber **7** and second measure chamber **10** and backward to the corresponding storing chamber (**4**, **5**, **6**).

The portable system **1** may comprise an outlet **12** and the pumping means may be configurated to evacuate the liquid sample from the fluidic circuit throughout said outlet **12.** Alternatively, the pumping means may be further configured to transport the fluid sample from the first measure chamber **7** and second measure chamber **10** backwards to the sampling module **2** evacuating the fluid sample by means of the inlet **11.**

### APPARATUS

The present invention further relates to a measurement apparatus for sampling and physicochemical analysis of a fluid, comprising at least one portable system **1** according to anyone of the embodiment described hereabove, and at least one extracting means **16** to extract a fluid sample from a soil **15.** The measurement apparatus comprises a junction element **14** for exchanging a liquid. The at least one portable system **1** and the at least one extracting means **16** are in fluidic communication.

According to one embodiment, the inlet **11** comprised in the sampling module **2** is in fluidic communication with a collection tube of the extracting means **16.**

According to one embodiment, the measuring apparatus comprises a frame comprising at least one first cavity for receiving at least one portable system **1** and at least one second cavity for receiving at least one extracting means **16.**

According to one embodiment, the measurement apparatus frame has cylindrical shape. The frame may be configured to be inserted in the soil **15.**

According to one embodiment, the extracting means **16** is a lysimeter. The extracting means **16** may be a suction lysimeter, simple chamber lysimeter, dual chamber lysimeter, pan lysimeter, static tension lysimeter, controlled tension lysimeter or a weighting lysimeter. The lysimeter used may be configure to collect a water same at a depth ranging from 10 cm to 2 m.

According to one embodiment, the measurement apparatus comprises multiple portable systems **1** fluidly connected to one extracting means inserted in the soil **15.**

According to one embodiment, the measurement apparatus comprises one portable systems **1,** comprising multiples cartridges, which is in fluidly connected to one extracting means **16** inserted in the soil **15.**

According to one embodiment, the measurement apparatus comprises multiple portable systems **1** fluidly connected one by one to extracting means **16** inserted in the soil **15** at different depth.

According to one embodiment, the measurement apparatus comprises one portable systems **1** where the inlet **11,** by means of a distributor, is put in fluid communication to multiples extracting means inserted in the soil **15** at different depth.

### COMMUNICATION

The portable system **1** according to the present invention, further comprises a communication module capable of transmitting information to a data processing system. According to one embodiment, the communication module is configured to further communicate with the lysimeter **16.** The communication module may comprise a near field communication device for short range communication, wireless communication, Bluetooth, wi-fi, 3G, or a Low-Power Wide-Area Network.

According to one embodiment, the portable system **1** further comprises a power unit. Said power unit may be a transformer connected with a voltage ranging from 110 V to 220 V.

According to one embodiment, the portable system **1** further comprises at least one computer printed board in communication with the communication module, the sampling means **2,** the physicochemical sensors **8,** the pumping means, and the distribution module **9.** The computer printed board may be configured to control the sampling means **2,** the physicochemical sensors **8,** the pumping means, and the distribution module **9** according to the user input received through the communication module. The computer printed board may be programmed to command the collection of a liquid sample and measurements, or to command the repetition of those two steps multiple times.

### METHOD

The invention also relates to a method for sampling fluid through extraction from a soil and physicochemical analyzing said fluid sample by the portable system 1 as described hereabove.

According to one embodiment, the method comprises a first step of calibration of the physicochemical sensors **8** of the portable system **1,** before the fluid sampling and analysis. The calibration step may be carried out by obtaining at least two calibration points for each of the physicochemical sensors **8** comprised in the first measure chamber **7** and/or second measure chamber **10.** According to this embodiment, at least a portion of the first calibration liquid is transferred from the first storing chamber **4** to the first measure chamber **7** and the second measure chamber **10.** A measurement of the concentration of the first calibration liquid, known by the user, is obtained for the physicochemical sensors **8** configured to perform chemical analysis. According to this embodiment, the first calibration liquid is then transferred backward to the first storing chamber **4** in order to empty the first measure chamber **7** and the second measure chamber **10.** The calibration point is obtained as the measured concentration of the first calibration liquid versus the real concentration of the first calibration liquid. Before proceeding to the following measure with the second calibration liquid, the fluidic circuit may be cleaned by connecting the third storing chamber **6** filled with the cleaning liquid to the first measure chamber **7** and the second measure chamber **10.** This would prevent any contamination of the second calibration liquid with the residual first calibration liquid that could falsify the concentration measure of the second calibration liquid concentration. The second calibration point may be obtained by fluidly connecting the second storing chamber **5** to the first measure chamber **7** and the second measure chamber **10,** and measuring the concentration of the second calibration liquid. The measured concentration of the second calibration liquid versus its real concentration gives the second calibration point. According to the embodiment described hereabove, wherein the portable system **1** comprises storing chambers additional to the first and the second storing chamber (**4**, **5**), additional calibration points are obtained for each of the additional storing chambers.

According to one embodiment, the calibrating step is followed by a step of cleaning the fluidic circuit by connecting the third storing chamber filled **6** filled with the cleaning liquid to the first measure chamber **7** and the second measure chamber **10.** The cleaning step would prevent any contamination of the liquid sample with the residual second calibration liquid that could falsify the physicochemical analysis of the liquid sample.

According to one embodiment, the method comprises a further step of collecting a fluid sample from the collecting tube of the lysimeter **16** with sampling pumping means of the sampling module **2** and transferring the fluid sample through the communication tube **14** to the inlet **11.** In others embodiments, the communication tube may be a junction element insuring exchanging fluid from the lysimeter **16** to the portable system **1.** The junction element may be, for example, a valve or a plurality of tubes or an aperture between two tanks or reservoirs, one being in the lysimeter **16** and the other in the portable system 1.

The method according to the present invention finally comprises the step of measuring at least one physiochemical parameter of the fluid sample. By means of non-limiting example the physicochemical parameter measured may be the pH, the ionic concentration of a chemical species such as the K⁺, Na⁺, Ca²⁺, Mg²⁺, Cl⁻, NH₄⁺, NO₂⁻, NO₃⁻, PO₄³⁻ and the like, or the conductivity.

According to one embodiment, the fluid sample is evacuated from the fluid circuit through the outlet **12.** According to an alternative embodiment, the fluid sample is evacuated from the fluid circuit through the inlet **11.** According to one embodiment, the evacuation of the fluid sample is further followed by a step of cleaning up of the fluidic circuit by connecting the third storing chamber filled **6** filled with the cleaning liquid to the first measure chamber **7** and the second measure chamber **10.**

According to one embodiment, the method further comprises the step of transmitting the information concerning the at least one measured physiochemical parameter of the fluid with the communication module to the data processing system.

According to one embodiment, the method of the invention is a standard addition method. According to one embodiment, the method of the invention is a dilution method. According to one embodiment, the method of the invention is a dilution method for calibration. According to one embodiment, when the method is a dilution method, the fluid sample is mixed to calibration solutions, preferably before to be rejected from the portable system **1** of the invention.

### INFORMATIC PRODUCT

The invention further relates to a computer implemented method configured communicate and command the portable system **1.** According to one embodiment, the computer implemented method is configured to receive, thanks to the communication module, the information collected by the portable system. According to one embodiment, the computer implemented method is configured to transmit information to the portable system, where said transmitted information comprises acquisition parameters defined by the user. The acquisition parameter may comprise the frequency of the measurements that may be performed each 1 minute, 2 minutes, 3 min, 3.5 min, 4 min, 4.5 min, 5 min, 5.5 min, 6 min, 6.5 min, 7 min, 7.5 min, 8 min, 8.5 min, 9 min, 9.5 min, 10 min, 11 min, 12 min, 13 min, 14 min, 15 min, 16 min, 17 min, 18 min, 19 min, 20 min, 21 min, 22 min, 23 min, 24 min, 25 min, 26 min, 27 min, 28 min, 29 min, 30 min, 31 min, 32 min, 33 min, 34 min, 35 min, 36 min, 37 min, 38 min, 39 min, 40 min, 41 min, 42 min, 43 min, 44 min, 45 min, 46 min, 47 min, 48 min, 49 min, 50 min, 51 min, 52 min, 53 min, 54 min, 55 min, 56 min, 57 min, 58 min, 59 min, 1h, 6h, 12h, 18h, 24h, 30h, 36h, 42h, 48h, 54h, 60h, 66h, 72h, 78h, 84h, 90h, 96h, 102h, 108h, 114h, 120h, 126h, 132h, 138h, 144h, 150h, 156h, 162h, 168h, 174h, 180h, 186h, 192h, 198h, 204h, 210h, 216h, 222h, 228h, 234, 240h, 246h, 252h, 258h, 264h, 270h, 276h, 282h, 288h, 294h, 300h, 306h, 312h, 318h, 324h, 330h, 336h, 342h, 348h, 354h, 360h, 366h, 372h, 378h, 384h, 390h, 396h, 402h, 408h, 414h, 420h, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, or 30 days.

According to one embodiment, the computer implement method is configured to program the printed computer board in order to perform the measurements according to the received user input.

According to one embodiment, the computer implemented method further comprises a step of displaying the information received from and transferred to the portable system **1.**

According to one embodiment, the computer implemented method comprises the steps that when executed causes the portable system **1** to perform a measurement or the calibration of the physicochemical sensors **8.**

According to one embodiment, the computer implemented method is configured to monitor and command the lysimeter **16** to perform the extraction of soil fluid to transfer to the portable system **1.**

According to one embodiment, the computer implemented method comprises the steps that when executed causes the portable system **1** to perform the dilution method for calibration.

According to one embodiment, the computer implemented method comprises the steps that when executed causes the portable system **1** to perform the dilution method.

According to one embodiment, the computer implemented method comprises the steps that when executed causes the portable system **1** to perform the standard addition method.

The present invention further relates to a computer program product for communicate and command the portable system **1,** the computer program product comprising instructions which, when the program is executed by a computer, cause the computer to carry out the steps of the computer-implemented method according to anyone of the embodiments described hereabove.

The present invention further relates to a computer-readable storage medium comprising instructions which, when the program is executed by a computer, cause the computer to carry out the steps of the computer-implemented method according to anyone of the embodiments described hereabove.

Yet another aspect of the present invention relates to a data processing system comprising means for carrying out the steps of the computer-implemented method according to anyone of the embodiments described hereabove in relation with the computer implemented method.

According to one embodiment, the data processing system is a dedicated circuitry or a general purpose computer, configured for receiving the data and executing the operations described in the embodiment described above concerning the computer implemented method. According to one embodiment, the data processing system comprises a processor and a computer program. The processor receives information concerning the physicochemical sensors **8** and processes said information under the instructions of the computer program. According to one embodiment, the computing device comprises a network connection enabling remote implementation of the method according to the present invention.

While various embodiments have been described and illustrated, the detailed description is not to be construed as being limited hereto. Various modifications can be made to the embodiments by those skilled in the art without departing from the true spirit and scope of the disclosure as defined by the claims.

### BRIEF DESCRIPTION OF THE DRAWINGS

**Figure 1** is a schematic representation of the fluidic circuit of the portable system **1.**
**Figure 2** is a schematic representation of the fluidic circuit of the portable system **1** according to the embodiment where the fluidic circuit further comprises a third storing chamber **6** and a second measure chamber **10.**
**Figure 3** is a schematic representation of the portable system **1** comprising a communication tube **14** configured to put in fluidic communication the portable system **1** and the lysimeter **16.**

### REFERENCES

- 1 -: Portable system;
- 2 -: Sampling module;
- 3 -: Disposable and removable cartridge;
- 4 -: First storing chamber;
- 5 -: Second storing chamber;
- 6 -: Third storing chamber;
- 7 -: First measure chamber;
- 8 -: Physicochemical sensor;
- 9 -: Distribution module;
- 10 -: Second measure chamber; 11 - Inlet;
- 12 -: Outlet;
- 13 -: Light source;
- 14 -: Communicating tube;
- 15 -: Soil;
- 16 -: Lysimeter.

## Claims

1. A portable system (1) for sampling and physicochemical analysis of a fluid, comprising a fluidic circuit, wherein the fluidic circuit comprises:
- a sampling module (2) configured to collect a sample of a fluid extracted from a soil (15);
- a disposable and removable cartridge (3) comprising a first storing chamber (4) filled with a first calibration liquid and a second storing chamber (5) filled with a second calibration liquid and further comprising a first measure chamber (7) comprising at least one disposable physicochemical sensor (8):
- a distribution module (9) in fluid communication with the sampling module (2) and the disposable and removable cartridge (3), wherein said distribution module (9) is capable of fluidly connect the first measure chamber (7) to one of the storing chambers (4, 5) or to the sampling module (2); and
- a pumping means to provide a flow from the sampling module (2) to the first measure chamber (7) and a flow from one of the storing chambers (4, 5) to the first measure chamber (7).

2. The portable system (1) according to claim **1,** wherein the cartridge further comprises a third storing chamber (6) comprising cleaning liquid and being in fluid communication with the distribution module (9); wherein the distribution module (9) is capable of fluidly connecting the first measure chamber (7) with said third storing chamber (6).

3. The portable system (1) according to anyone of claims **1** to **2,** further comprises a second measure chamber (10) comprising at least one physicochemical sensor (8) and being in fluid communication with the sampling module (2) and the distribution module (9); wherein the distribution module (9) is capable of fluidly connect the second measure chamber (10) with one of the storing chambers (4, 5, 6).

4. The portable system (1) according to anyone of claims **2** to **3,** wherein the first storing chambers (4), the second storing chamber (5) and the third storing chamber (6) are fluidly isolated from each other.

5. The portable system (1) according to anyone of claims **1** to **4,** wherein the sampling module comprises an inlet (11) configured to be in fluid communication with at least one collection tube of a lysimeter (16).

6. The portable system (1) according to anyone of claims **1** to **5,** wherein the pumping means are fluidly connected to the sampling module (2), ensuring a fluid flow in at least fluidic circuit, the first measure chamber (7) and the second measure chamber (10).

7. The portable system (1) according to anyone of claims **1** to **6,** wherein pumping means are fluidly connected to the storing chambers (4, 5, 6) and capable of providing a flow from the storing chambers (4, 5, 6) to the first measure chamber (7) and second measure chamber (10) and backwards.

8. The portable system (1) according to anyone of claims **1** to **7,** wherein the first calibration liquid and the second calibration liquid are solutions comprising a same chemical species with a significantly different concentration.

9. The portable system (1) according to anyone of claims **3** to **8,** wherein the at least one physicochemical sensor (8) comprised in the second measure chamber (10) is an optical sensor or an electrochemical sensor.

10. The portable system (1) according to anyone of claims **1** to **9,** wherein said at least one physicochemical sensor (8) comprised in the second measure chamber (10) is adapted to monitor the conductivity, the oxidation-reduction potential, the temperature of the fluid, chemical oxygen demand, the carbon dioxide concentration, total organic carbon and/or dissolved oxygen.

11. The portable system (1) according to anyone of claims **1** to **10,** further comprising a communication module capable of transmitting information to an interface device.

12. The portable system (1) according to anyone of claims **1** to **11,** wherein the pumping means is a peristaltic pump.

13. The portable system (1) according to anyone of claims **1** to **12,** wherein the sampling module (2) further comprises a UV emitting light source (13).

14. A measurement apparatus for sampling and physicochemical analysis of a fluid, comprising at least one portable system (1) according to anyone of claims **1** to **13,** and at least one extracting means (16) to extract a fluid sample from a soil (15), where the measurement apparatus comprises a junction element (14) for exchanging a liquid.

15. A measurement apparatus according to claim **14,** where comprises a frame comprising at least one first cavity for receiving at least one portable system (1) and at least one second cavity for receiving at least one extracting means (16).
